# EUROPEAN PATENT APPLICATION

(11) **EP 1 457 217 A1**
(43) Date of publication of application: **15.09.2004**
(21) Application number: 04004804.3
(22) Date of filing: 02.03.2004
(51) Int. Cl.: A61M 1/06, A61M 1/00

(54) **Electric motor-operated breast pump for personal use**

(30) Priority: 14.03.2003 IT MI20030490
(71) Applicant: ARTSANA S.p.A., 22070 Grandate, (Como) (IT)
(72) Inventor: Catelli, Pietro, 22100 Como (IT)
(74) Representative: Ripamonti, Enrico, Dr. Ing.

(57) **Abstract**

A breast pump (1) for personal use comprises a member (6) generating an alternating sucking effect within a cup part (3) disposed on a mother's breast, said suction member (6) being subjected to an actuator element (57) driven by an electric motor (13). The suction member (6) comprises a rigid body (10) movable axially within an undeformable containing body (11) removably coupled to a support (12) with which the electric motor (13) is associated, the actuator element (57) operationally cooperating with the movable body (10) in order to move it and induce an alternating suction effect within the cup part (3) associated with the mother's breast.

## Description

The present invention relates to an electric motor-operated breast pump for personal use, in accordance with the introduction to the main claim.

Manual and electric motor-operated breast pumps have been known for some time. Both these types comprise a sucking enclosure with a funnel or cup portion to be coupled to a mother's breast and connected to a container for the milk extracted from the breast and to a suction member. This latter is generally a piston movable within a cylinder to which the sucking enclosure is operationally connected via a suitable channel. The piston can be operated manually (manual breast pump) or automatically by an actuator operated by an electric motor (electric motor breast pump).

Breast pumps are also known in which the manually operable suction member of cylinder and piston type is removably associated with the sucking enclosure and can be connected to an operating unit with electric motor to achieve automatic extraction of the milk from the breast. This type of breast pump also enables several persons to use a motor-operated breast pump in which there is only one operating unit, but in which the sucking enclosure and the suction member (cylinder and piston) is personal. In other words, the state of the art comprises an automatic motor-operated breast pump in which the cylinder-piston suction member and the sucking enclosure connected to it are of personal use in the sense that other suction members pertaining to other persons can be connected to said motor in substitution of said suction member or in parallel with it. This enables several persons to use breast pumps presenting a common operating or motor unit which is used by different women to extract the mother's milk, and a sucking enclosure and suction member personal to each user. This arrangement is particularly advantageous from the hygiene and sanitary viewpoint in those cases in which several women have to use the breast pump.

The aforedescribed known arrangement is however of complicated construction because of the facility for using the suction member either manually or automatically.

An object of the present invention is to provide a breast pump in which the operating part can be used for a plurality of different suction members connected to corresponding suction bodies to enable personal use of the breast pump, said breast pump being of simple construction and use.

Another object is to provide a breast pump of the stated type in which the different suction members can be quickly and easily coupled to or separated from the operating part.

These and further objects which will be apparent to the expert of the art are attained by a breast pump in accordance with the accompanying claims.

The present invention will be more apparent from the accompanying drawing, which is provided by way of nonlimiting example and in which:
Figure 1 is a perspective view of a breast pump of the invention seen from above;
Figure 2 is a perspective view of the breast pump of Figure 1 seen from below;

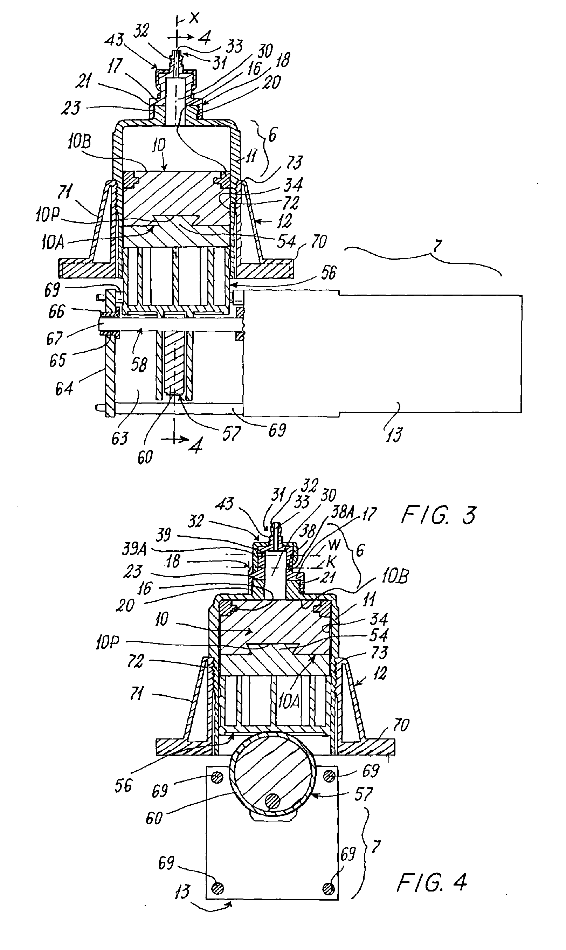

Figure 3 is a section on the line 3-3 of Figure 1;
Figure 4 is a section on the line 4-4 of Figure 3, but with the breast pump in a different stage of use;
Figure 5 is a side view of a suction member removed from the breast pump of Figure 1;
Figure 6 is a perspective view of a first part of the member of Figure 5 seen from below; and
Figure 7 is a side view of a second part of the member of Figure 5.

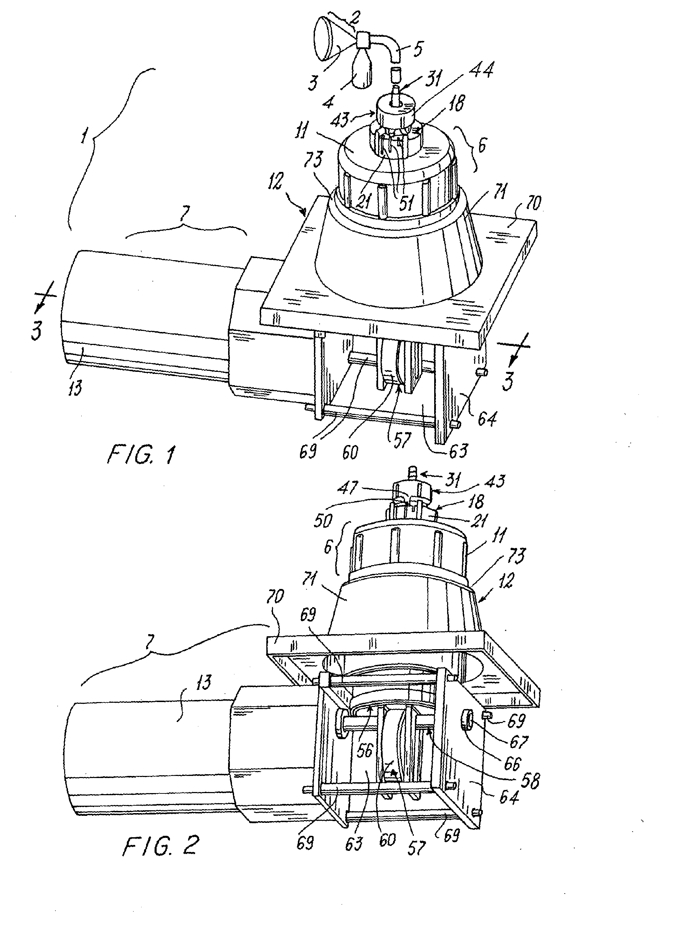

With reference to said figures, a breast pump is indicated overall by 1 and comprises: a known sucking enclosure 2 for the mother's milk, having a funnel or cup part 3 to be coupled to a breast, a bottle 4 removably coupled to said enclosure 2, a conduit 5 connecting the enclosure 2 to a suction member 6 and a motor-driven operating unit 7 enabling the milk to be withdrawn from the breast automatically (i.e. not manually). The enclosure 2, the bottle 4 and the conduit 5 are known components and are shown in Figure 1 schematically and not to scale.

The suction member 6 comprises a rigid body 10 movably inserted into an undeformable body 11 removably associated with a support 12 to which an electric motor 13 is fixed, the support and motor defining the operating unit 7. More particularly, the movable body 10 comprises a dovetail-shaped lower part 10A and a free end part 10B carrying an annular lateral seal element 16. This annular element is located in a lateral groove 10K provided in the rigid body 10.

The undeformable body 11 presents an end part 17 with which an element 18 is associated for connection to the conduit 5. This element comprises a portion 20 inserted into the part 17, and a portion 21 (concentric with the portion 20) mounted and screwed onto the outside of an end part 23 of the undeformable body 11.

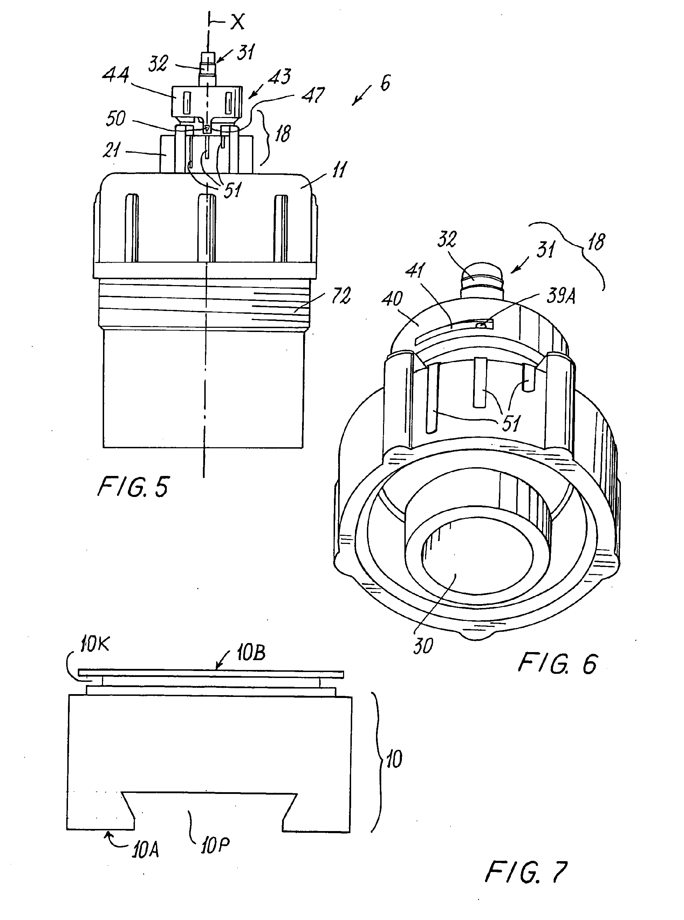

The connection element 18, hollow internally at 30, comprises (see Figure 6) a terminal part 31 with an outer peripheral stepped surface 32. The part 31 is holed axially and therefore presents a cavity 33 connected to the cavity 30 of the connection element 18 which, in its turn, is connected to a cavity 34 of the undeformable body 11 within which the body 10 moves. The stepped surface 32 facilitates reliable and secure connection of the conduit 5 to the suction member 6.

In the part 31 there are provided two conduits 38 and 39 having axes K and W perpendicular to the longitudinal axis X of the suction member 6. These conduits open, at 38A and 39A, into the surface 40 of the portion 21 of the connection element 18. The conduit 38 enables air pressure to be discharged to the outside of the cavity 33, and which would otherwise be fed to the breast when the movable body 10, during operation of the breast pump, moves to the upper end of the cavity 34 of the undeformable body 11 (in proximity to the cavity 30), so compressing the air present in it. This avoids obvious discomfort to the woman. The conduit 39 opens at 39A within a surface recess 41 provided in the surface 40 of the portion 21, and having a variable depth with its greatest depth in correspondence with the opening 39A; the suction pressure on the milk present in the conduit 5 can be varied by throttling the conduit 39.

To achieve the aforesaid pressure controls, on the connection element 18 there is mounted a pressure regulator member 43. This comprises a socket-shaped piece 44, of elastically deformable material such as silicon rubber or the like, open on one side through which the part 31 passes and presenting an edge 45 from which two tabs (only one, 47, being shown in the figures) of different dimensions project. The tab of larger dimensions but of smaller thickness that the tab 47 is arranged to lie in correspondence with the conduit 38.

Because of its reduced thickness, this tab is able to withdraw from the connection element 18 during the compression stage of the movable body 10, enabling the pressure formed in the cavity 33 to be discharged through the conduit 38.

In contrast, the tab 47 is arranged to lie on the recess 41 in correspondence with the conduit 39. Depending on its position on said recess or conduit, the suction pressure exerted in the conduit 5 by the movable body 10 is either reduced or not; shut-off of the conduit 39 results in maximum suction pressure, whereas if the tab 47 is positioned at the smallest-depth part of the recess 41 the conduit 39 is opened to allow greater air flow into the cavity 33 from the outside, so reducing the suction pressure in the conduit 5.

The tab 47 carries a reference marker 50 arranged to cooperate with notches 51 provided in the portion 21 of the connection element 18; in this manner the different suction pressures obtainable by moving the pressure regulator member 43 about the X axis can be displayed.

As stated, the movable body 10 comprises a dovetail-shaped lower part 10A (i.e. with a dovetail-shaped recess 10p).

This lower part 10p is arranged to cooperate with a projecting upper part 54 of a presser member 56 freely movable within the cavity 34 of the undeformable body 11 and subjected to the action of a cam element 57 keyed eccentrically onto an output shaft 58 of the known electric motor 13. Preferably, the cam element 57 presents a yieldable peripheral element 60 arranged to cooperate with the presser member 56, said yieldability ensuring silent coupling with this latter.

The projecting part 54 of the presser member is removably connectable to the lower part 10A of the body 10, and in particular is removably insertable into the said dovetail recess 10p, to hence connect the member 56 to the body 10 in a separable manner.

The cam element 57 moves within a space 63 defined between the motor 13 and a plate 64 presenting a hole 65 containing a bush 66 supporting the free end 67 of the output shaft 58. The plate is fixed to the casing of the motor 13 by rods 69.

The support 12 is rigid in known manner with the casing of the motor 13 and with the plate 64. This support comprises a flat part 70 with a raised hollow portion 71 (frusto-conical in the figures) into which the undeformable body 11 is inserted. This latter has a threaded portion 72 removably screwed into one end 73 of the hollow portion 71 of the support 12. As will be described, this enables the undeformable body 11 with the connected movable body 10 (i.e. the suction unit 6) to be easily separated from the operating unit 7 and be replaced by another suction unit connected to a different milk sucking enclosure 2. This therefore allows the breast pump to be easily and hygienically used by a number of women each provided with her own sucking enclosure 2 (and bottle 4).

The use of the breast pump is apparent from the aforegoing description: on operating the motor 13, the rotation of the shaft 58 causes the cam element 57 to move, within the cavity 34, towards and away from the connection element 18, with reciprocating movement, the movable body 10 hence producing suction on the milk of the mother's breast within the sucking enclosure 2.

When a different woman wishes to use the breast pump, the undeformable body 11 is separated from the operating unit 7 and the body 10 is separated from the presser member 56. A different body 10 and a second undeformable body (with the described components associated therewith) personal to the different user can now be coupled to the unit 7 and used to extract milk from the breast.

A preferred embodiment of the invention has been described. Other embodiments are however possible: for example the presser member 56 can be in one piece with the cam element 57, or the movable body 10 can be removably coupled to the member 56 in a manner different from that described, for example by a bayonet or other connection (for example by elastic tangs) which enables the parts to be easily separated; alternatively the annular lateral element 15 can be dispensed with, and the body 10 cooperate with minimum clearance with the wall of the cavity 34 in which it is positioned. These variants are also to be considered as falling within the scope of the present invention.

## Claims

1. A breast pump for personal use comprising a member (6) generating an alternating sucking effect within a cup part (3) disposed on a mother's breast, said suction member (6) being subjected to an actuator element (57) driven by an electric motor (13), **characterised in that** the suction member (6) comprises a rigid body (10) movable axially within an undeformable containing body (11), this latter being removably coupled to a support (12) with which the electric motor (13) is associated, the actuator element (57) cooperating with a presser member (56) removably associated with the movable body (10) in order to move this latter within the undeformable body and induce an alternating suction effect within the cup part (3) associated with the mother's breast.

2. A breast pump as claimed in claim 1, **characterised in that** the presser member (56) is coupled to the movable body (10) by a separable mechanical dovetail coupling.

3. A breast pump as claimed in claim 1, **characterised in that** the presser member (56) is coupled to the movable body (10) by a separable bayonet coupling.

4. A breast pump as claimed in claim 1, **characterised in that** the presser member (56) is coupled to the movable body (10) by a releasable elastic connection.

5. A breast pump as claimed in claim 1, **characterised in that** the undeformable containing body (11) is socket-shaped and is positioned above the actuator element (57).

6. A breast pump as claimed in claim 1, **characterised in that** the undeformable containing body (11) opens laterally, the actuator element (57) penetrating laterally into said body (11).

7. A breast pump as claimed in claim 1, **characterised in that** the presser member (56) is rigid with the actuator element (57).

8. A breast pump as claimed in claim 1, **characterised in that** the undeformable containing body (11) is coupled to the support (12) by screwing.

9. A breast pump as claimed in claim 1, **characterised in that** the undeformable containing body (11) is bayonet-coupled to the support (12).

10. A breast pump as claimed in claim 1, **characterised in that** the undeformable containing body (11) comprises a part (23) of reduced cross-section on which there is mounted and secured a connection element (18) presenting an internal cavity (30), and a part (31) removably connected to a conduit (5) connecting the suction member to a sucking enclosure (2) for the mother's milk provided with the cup part (3).

11. A breast pump as claimed in claim 10, **characterised in that** the connection element (18) presents holes (38, 39) opening (at 38A, 39A) into the surface (40) of said element (18), said holes having their axis (K, W) perpendicular to the longitudinal axis (X) of the suction member (6), said holes communicating with the internal cavity (30) of said connection element (18), said holes (38, 39) cooperating with a pressure regulator member (43) arranged to modify the suction pressure on the milk and positioned on the connection element (18), one of said holes (39) opening into a variable-depth surface recess (41) in said element (18).

12. A breast pump as claimed in claim 11, **characterised in that** the pressure regulator member comprises a socket-shaped piece (44) mounted about the connection element (18) and presenting a free edge (45) from which there project tabs (47) arranged to cooperate with the holes (38, 39) in said element (18).

13. A breast pump as claimed in claim 12, **characterised in that** the tabs (47) of the connection element (18) have different dimensions.

14. A breast pump as claimed in claim 13, **characterised in that** one of the tabs of the connection element (18) is of deformable material.

15. A breast pump as claimed in claim 13, **characterised in that** one (47) of the tabs of the connection element (18) is positioned in correspondence with the surface recess (41) of said connection element (18) where a hole (39) of this latter opens, and is arranged to reduce the flow of air into the cavity (30) of said element (18) through said hole in order to vary the suction pressure on the mother's milk.

16. A breast pump as claimed in claim 1, **characterised in that** the actuator element comprises a cam (57) positioned on the output shaft (58) of the electric motor (13).

17. A breast pump as claimed in claim 16, **characterised in that** the cam element (57) is rigid with the presser member (56) to which this latter is fixed.

18. A breast pump as claimed in claim 16, **characterised in that** the cam element is in one piece with the presser member (56).

19. A breast pump as claimed in claim 13, **characterised in that** the cam element (57) is covered on its surface by a yieldable annular element (60).

20. A breast pump as claimed in claim 1, **characterised in that** the movable body (10) carries an annular seal element (16).
